# EUROPÄISCHE PATENTSCHRIFT

(11) **EP 0 679 626 B1**
(45) Veröffentlichungstag und Bekanntmachung des Hinweises auf die Patenterteilung: **11.11.1998**
(21) Anmeldenummer: 95105091.3
(22) Anmeldetag: 05.04.1995
(51) Int. Cl.: C07C 37/74, C07C 39/16, C08G 64/00

(54) **Verfahren zur Herstellung von ultrareinem Bisphenol-A und dessen Verwendung**
Process for the preparation of ultrapure bisphenol-A and use thereof
Procédé de préparation de bisphénol-A très pur et son emploi

(30) Priorität: 18.04.1994 DE 4413396
(43) Veröffentlichungstag der Anmeldung: 02.11.1995
(73) Patentinhaber: BAYER AG, 51368 Leverkusen (DE)
(72) Erfinder: Wulff, Claus, Dr., D-47800 Krefeld (DE); Hallenberger, Kaspar, Dipl. Ing., D-51375 Leverkusen (DE); Steude, Heinrich, Dipl. Ing., D-51375 Leverkusen (DE); Meurer, Kurt-Peter, Dr., B-2000 Antwerpen (BE); van Osselaer, Tony, Dr., B-9111 St. Niklaas/Belsele (BE); Hinz, Jürgen, Dr., B-2930 Brasschaat (BE); Quaeyhaegens, Frank Dr., B-2640 Mortsel (BE); Vaes, Johan, Dipl.Ing., B-2920 Kalmthout (BE); Hooftman, Ignace Dipl.-Ing., B-9150 Kruibeke (BE)

(56) Entgegenhaltungen:
- EP-A- 0 290 179
- EP-A- 0 499 922
- EP-A- 0 523 931
- EP-A- 0 567 855

## Beschreibung

Die Erfindung betrifft ein Verfahren zur Herstellung von ultrareinem Bisphenol-A (p,p-BPA-Gehalt > 99,98%). Schwersiedende Isomere wie Mol 402, Trisphenol, Spiroindan, Indan etc., Metallen, Metallabrieb, Metall-/Alkali-Metallionen, Salze, saure Bestandteile, Oligo-/Polymere, gegebenenfalls funktionalisiert, sowie tensidaktive Verbindungen werden abgetrennt.

Bisphenol-A (BPA) wird z.B. nach bekannten Verfahren aus Aceton und Phenol in Gegenwart von gegebenenfalls modifizierten Ionenaustauscherharzen hergestellt (z.B. DE-A 3 727 641, entsprechend US-A 4 912 263). So hergestelltes Bisphenol-A ist bereits ohne weitere Reinigung als ein Monomerbaustein zur Herstellung von anderen Polymeren, z.B. Polycarbonaten, geeignet.

Sollen jedoch die mit Bisphenol-A hergestellten Polymeren besondere Reinheiten aufweisen und damit hohen Anforderungen genügen, so muß bereits bei einer Herstellung in hoher Reinheit anfallendes Bisphenol-A weiter gereinigt werden, z.B. durch Kristallisation eines Bisphenol-A/Phenol-Adduktes und anschließendes Entfernen des Phenols (z.B. DE-A 4 105 428, DE-A 4 213 872). Methoden zur destillatiren Entfernung von Phenol aus BPA/Phenol-Addukten gehen aus EP-A 290 179 und EP-A 523 931 hervor.

So hochgereinigtes Bisphenol-A hat eine Reinheit von 99,60 bis 99,90% p,p-BPA-Gehalt und eine Hazen-Farbzahl von 20 (Schuppenware). Der Test nach Hazen bei 166°C, 1,5h lang gemessen, ergibt eine Farbzahl von 50.

Es wurde nun gefunden, daß hochreines Bisphenol-A mit weit besserer Farbzahl (Hazen 0-5) gewonnen werden kann, wenn es im Vakuum fraktioniert und bei einer sukzessiven Abtrennung von Schwer-, Leichtsiedern und nicht destillierten Bestandteilen, destilliert wird.

Gegenstand der Erfindung ist ein Verfahren zur Herstellung von ultrareinem Bisphenol-A mittels kontinuierlicher oder diskontinuierlicher Destillation, wobei bei diskontinuierlicher Destillation als Kolonnenkopf ein modifizerter Flüssigteiler eingesetzt wird, dadurch gekennzeichnet, daß
(a) eine Bisphenol-A-Schmelze, hergestellt nach üblichen BPA-Herstellungsprozessen oder durch Aufschmelzen von BPA-Schuppen-, Pulver- oder - Prillware, mit einem Gehalt an p,p-Bisphenol-A von mindestens 90% und Phenolgehalten <10% als Edukt eingesetzt und destilliert wird,
(b) die Destillation dieser Schmelze ohne Stabilisatorzusatz unter inerten Bedingungen durchgeführt wird,
(c) die Destillation so geführt wird, daß die Verluste durch Zersetzung von p,p-Bisphenol-A <0,5% betragen,
(d) die Destillation bei Temperaturen von 220°C-250°C durchgeführt wird,
(f) die Destillation unter Vakuum bei 1-50 mbar erfolgt,
(g) die Destillation zweistufig erfolgt,
(h) zuerst
   (h,1) Schwersiederanteile wie Mol 402, Indane und Trisphenole vollständig abgetrennt werden, dann
   (h,2) Metallabrieb, Metallionen- und Salzspuren, Säurespuren, poly-/oligomere Katalysatorausschwemmungen und nicht destillierbare andere höhermolekulare Verbindungen vollständig abgetrennt werden, dann
   (h,3) tensidaktive Verbindungen, vollständig abgetrennt werden, dann
   (h,4) leichtsiedende Anteile wie o,p-BPA, Alkylphenole, Phenolspuren sowie schwefelhaltige Leichtsieder wie Mercaptopropionsäure, abgetrennt werden, dann
   (h,5) mit Leichtsiedern, Phenol, Schwersiedern angereicherte BPA-Schmelzlösungen mit einem Gehalt an p,p-Bisphenol-A >92% als Rückströme in den Prozeß zurückgeführt werden,
(i) die Ausschleusung der Leichtsieder angereicherten BPA-Schmelzelösungen in den Prozeß nicht größer als 5% und die Ausschleusung der Schwersieder angereicherten BPA-Schmelzelösungen, nicht größer als 10% ist,
(j) Leichtsieder und Schwersieder in den mit p,p-BPA vermischten Ausschleuseprodukten aus den Rückströmen in den Prozeß zurückgeführt umgelagert und teilweise gewonnen werden,
(k) destilliertes p,p-Bisphenol-A als stabiles Sumpfprodukt lastunabhängig auf übliche Weise aufgearbeitet wird.

Das gereinigte ultrareine Bisphenol-A hat eine Reinheit >99,98% p,p-Bisphenol-A-Gehalt. Durch vollständige Abtrennung (<10 ppm) der Hauptverunreinigung o,p-BPA, der Schwersieder und nicht destillierbarer Komponenten aus dem BPA-Prozeß hat es eine Thermofarbstabilität in der Schmelze von Hazen bis zu kleiner 5 und zeichnet sich durch eine Thermo-Langzeit-Stabilität (>200 Stunden) der Schmelze aus.

Erfindungsgemäß wird vorgereinigtes BPA eingesetzt, wobei die Vorreinigung des Bisphenol-A zunächst über die Kristallisation von BPA-Phenoladdukten, dann durch Filtration der Addukte und anschließende Desorption des Phenols und dann die Destillation der BPA-Schmelze erfolgt.

Das Verfahren der Destillation zeichnet sich insbesondere dadurch aus, daß die Hauptverunreinigung im hergestellten Bisphenol-A, o,p-BPA, bis auf <10 ppm Gehalt reduziert werden kann. Die leicht siedenden Bestandteile werden auf <150 ppm reduziert.

Hochreines erfindungsgemäß destilliertes BPA unterscheidet sich von hochreinem kristallisiertem BPA dadurch, daß es in alkalischen Lösungen tensidinaktiv, besonders thermostabil ist und einen o,p-BPA-Anteil <10 ppm besitzt. Eine NaBPA-Lösung, die erfindungsgemäß destilliertes Bisphenol-A gelöst enthält, zeigt beim Schütteln oder bei Gaseinleitung (z.B. Durchperlen von N₂ bei Raumtemperatur) keine Schaumbildung mehr (hohe Oberflächenspannung).

Fraktioniert destillativ gereinigtes Bisphenol-A zeichnet sich zudem gegenüber dem aus Lösemitteln oder Phenol durch übliche, großtechnisch betriebene Umkristallisation erhaltenen Bisphenol A dadurch aus, daß die Schwersieder und nicht destillierbaren Bestandteile vollständig, o,p-Bisphenol A bis <10 ppm und Leichtsieder sowie Phenol <150 ppm abgetrennt sind.

Als Leichtsieder werden Stoffe genannt, die einen Siedepunkt geringer als Bisphenol A besitzen (p,p-BPA: 220°C, 5 hPa) wie z.B. o,p-BPA, Phenol, alkylierte Phenole, Chromane etc. Als Schwersieder werden Stoffe bezeichnet, die einen Siedepunkt höher als Bisphenol A besitzen, wie z.B. Mol 402, Trisphenol, Indan, Spironindan oder andere Verbindungen mit Molgewicht > 300.

Das erfindungsgemäß gereinigte p,p-Bisphenol-A kann als flüssiges, transportierbares und bei Temperaturen > 160°C lagerungsfähiges, thermostabiles Vorprodukt für Polymere gelagert werden.

Bei der erfindungsgemäßen Destillation wird Bisphenol-A mit einem Gehalt von mindestens 90 Gew.-% an Bisphenol-A und weniger als 10 % Isomeren aus der Schmelze heraus im Vakuum unter Inertgas fraktioniert destilliert. Die Destillation kann diskontinuierlich unter Einsatz einer Kolonne mit Flüssigkeitsteiler oder Gasteiler bzw. kontinuierlich durch sukzessive Abtrennung von Schwersieder und Leichtsieder mit Hilfe von zwei technisch ausgelegten Kolonnen durchgeführt werden.

Die kontinuierlich betriebene Zwei-Kolonnenfahrweise unter Vakuum wird so durchgeführt, daß zuerst Schwersieder, nicht destillierbare und Instabilitäten verursachende Bestandteile (Kolonnensumpf 1) und dann die Leichtsieder abgetrennt werden mit dem Vorteil, daß eventuell im Kolonnensumpf aus Schwersiedern und Bisphenol-A durch chemische Umlagerungs- oder Zersetzungsprozesse entstehende Leichtsieder in Kolonne 2 mit den im Edukt vorhandenen Leichtsieder über Kopf in Kolonne 2 abgetrennt werden können.

Der Druck beträgt 0,1 bis 10 mbar, die Temperatur im Sumpf der Kolonnen 220 bis 250°C.

Schwefelhaltige Schwersieder (oligomere Sulfate etc.), Überreste von Mercaptopropionsäure und andere schwefelenthaltende Leichtsieder werden so aus dem Bisphenol-A entfernt.

Das erfindungsgemäß gereinigte Bisphenol-A hat einen Gehalt von bis zu 99,99 Gew.-% Bisphenol-A, weniger (<) als 100 ppm Isomeren sowie eine Farbzahl nach Hazen kleiner (<) 5 (Thermostabilität bei 166°C: 1,5 Stunden). Das so gereinigte p,p-BPA ist bei 230°C mindestens 20h und bei 170°C mindestens 200 h farbstabil.

Ein bereits durch Kristallisation der Bisphenol-A/Phenol-Addukte vorgereinigtes, im erfindungsgemäßen Verfahren einsetzbare, Bisphenol-A enthält weniger (<) als 10 % Isomere und Phenol.

Das Ergebnis der hohen Reinheit in Verbindung mit der ausgezeichneten Farbzahl und insbesondere die Abtrennung schwer- und leichtsiedender Isomeren, nicht destillierbarer Bestandteile der großtechnisch hergestellten Bisphenol-A-Schmelze sowie die unerwartete hohe Farbthermostabilität war nicht vorhersehbar, da bekanntlich Bisphenol-A bei höheren Temperaturen zerfällt und isomerisiert. Dies galt bisher als großer Nachteil des Destillationsverfahrens von Bisphenol-A.

Destilliertes Bisphenol-A zeichnet sich stofflich gegenüber nach anderen Verfahren gereinigten Bisphenolen dadurch aus, daß ein sehr geringer Anteil an o,p-BPA (<10 ppm) vorliegt. Hierdurch wird destilliertes Bisphenol A als Einsatzprodukt in Schmelzkondensationen interessant. o,p-BPA gilt in Schmelzprozessen als Kettenabbrecher und farbverschlechternde Komponente (Zersetzungsneigung).

Hochreines Bisphenol-A, das aus (einem) Umkristallionsschritt (-schritten) aus Lösemitteln oder Phenol gewonnen wird, unterscheidet sich von hochreinem destilliertem Bisphenol-A dadurch, daß die Konzentration aller vorkommenden Isomerengehalte über die einzelnen Umkristallisationsschritte zwar verringert aber immer noch - wenn auch weniger - in der BPA-Schmelze vorhanden sind (<1 ppm bis 50 ppm über alle Isomeren), insbesondere o,p-Bisphenol A (> 20 ppm) als Hauptverunreinigungskomponente heutiger auf dem Markt gängiger, hochreiner Bisphenol-A-Produkte.

Eine großtechnische durchgeführte Umkristallisation von Bisphenol-A ist zudem technisch aufwendiger und im Vergleich mit der Destillation teuer. Die erheblichen Lösungsmittelbewegungen und die notwendigen Tankvolumina bei den Umkristallisationsverfahren ist ein weiterer Nachteil. Energetisch ergeben sich weiterhin Nachteile für eine Kristallisation.

Ein weiterer Vorteil der Destillation gegenüber einer diskontinuierlich betriebenen Kristallisation ist neben der geringeren Störanfälligkeit die kontinuierliche Fahrweise. Sie ermöglicht eine Reinheitskonstanz der BPA-Schmelze, d.h. die hohe Farbstabilität und Reinheit der Schmelze wirkt sich qualitätssteigernd bei Abschuppung oder Prillung sowie bei der Herstellung von Natriumbisphenolat-Lösungen aus, die wiederum die Qualitätskonstanz hochtransparenter, hochreiner Polymerer (Polykondensate) bestimmen.

Hochreiner Bisphenol-A-Rohstoff führt zudem zur Verringerung von farbstabilisierenden Additiven für farbsensitive Polymere, z.B. bestimmte Polycarbonat-Typen.

Ein weiterer Vorteil von hochrein destilliertem Bisphenol A gegenüber hochreinem, kristallisiertem Bisphenol A ist die schaumarme bis schaumfreie Auflösung zu Natriumbisphenolatlösungen (NaBPA), die in großen Mengen als Einsatzstoff in der Polycarbonatherstellung dienen. Das bedeutet, daß NaBPA-Lösungen, die destilliertes Bisphenol A enthalten, eine geringere Tensidaktivität, d.h. eine höhere Oberflächenspannung besitzen. Die Schaumbildungsaktivität dieser Lösungen, die großtechnisch bei notwendigen Inertisierungs-, Transport-(Pumpvorgängen bzw. Lagerung in Tanks und Rohrleitungen) sowie Verarbeitungsvorgängen (Reaktionsführung im Reaktor) entstehen, wird deutlich herabgesetzt. Stabile Emulsionen werden dadurch verhindert. Auch erreichen hochreine kristallisierte Bisphenole (99,90 %-99,98 % p,p-BPA) die extrem hohen Oberflächenspannungswerte destillierter, hochreiner Bisphenole in NaBPA-Lösungen nicht.

Weiterhin werden durch Kolonnensumpfabtrennung im Prozeß bisher notwendige Filtrationsvorgänge der BPA-Schmelze vermieden. (Abtrennung von Metallabrieb mit Gehalt an Fe, Ni, Cr). Da Bisphenol-A einmal überdestilliert wird (2-Kolonnen-Fahrweise: Kolonne 1) sind auch keine Mikroteilchen mehr in der p,p-BPA-Schmelze mehr aufzufinden.

Die Bilanzierung der Destillation ergibt folgendes Bild: Es werden nur sehr wenige umlagerbare Isomere bei der Destillation gebildet wie o,p-BPA oder Alkylphenole. Schwersieder zerfallen zugunsten von o,p-BPA, Phenol, Alkylphenolen. Der Verlust über den ganzen Prozeß an p,p-BPA beträgt <0,1%.

Das erfindungsgemäß durch Destillation gereinigte Bisphenol-A ist bei Temperaturen > 160°C lagerungsfähig und über lange Zeiträume stabil. Es kann daher in flüssiger Form gelagert und transportiert werden. Es kann ohne vorherige Abkühlung direkt zur Herstellung von anderen Polymeren, z.B. Polycarbonat, Polyimiden, Polyestern, Polyacrylaten, Polysulfonen, Polyetherketonen, Blockcopolymeren, Copolymeren usw. eingesetzt werden.

### Beispiele

### Labordestillation von Bisphenol A (diskontinuierliche Versuche) Experimentelle Bedingungen der Diskonti-Laborversuche

Es wurde in einer Doppelmantel-Glaskolonne von 1,2 m Länge (Innendurchmesser 50 mm), 0,5 m gefüllt mit Glasringen von d = 10 mm. Als Kolonnenkopf wurde ein Gasteiler bzw. ein Flüssigkteiler verwendet, der für die Kondensation von Leichtsiedern modifiziert wurde. Die Leichtsieder wurden durch Wasserkühlung ca. 200 mm oberhalb der Entnahme immobilisiert. Die Kondensation der Destillate erfolgte mit Glycerin bei 160°C.

In der Regel wurde bei einem Druck von ca. 0,5 mbar, gemessen am Kolonnen Ausgang, destilliert. Die Temperatur im Kolonnenkopf an der Entnahmestelle lag bei 200°C; die Dampfdruckkurve Bisphenol-A zugrunde gelegt, bedeutet dies einen Druck von ca. 1,5 mbar. Das Rückflußverhältnis wurde auf 1,5:1 eingestellt (Destillationszeit: <8 h). Der Energieeintrag erfolgte über eine Mantelbeheizung am Kolonnensumpf. Die gesamte Apparatur wurde vorab intensiv mit Stickstoff inertisiert. Während der Destillation wurde kontinuierlich Stickstoff durch eine Kapillare eingeblasen.

### Versuchsdurchführung der Diskonti-Laborversuche

Durchführung der fraktionierten Vakuum-Destillation der BPA-Schmelze wurde BPA-Schuppenmaterial (z.B. Ausgangsreinheit 99,72 % p,p-BPA-Gehalt) in einer Apparatur mit konventionellem Gasteiler-Kopf destilliert.

Bei einem Rückflußverhältnis von 2:1 und einem Druck von 1 bis 1,5 mbar wurden zwei Vorlauffraktionen von ca. 10 % und 20 % abgenommen; ca. 50 % wurden als Hauptfraktion mit 99,95 % bis 99,96 % p,p-BPA-Gehalt isoliert.

Eine bessere Destillat-Qualität konnte mit einem Gasteiler nicht erreicht werden; Leichtsieder und das Dimer von Isopropylidenphenol konnten im Kolonnenkopf nicht ausreichend immobilisiert werden und gelangten ins Destillat.

Erheblich verbesserte Ergebnisse konnten mit einem modifizierten Flüssigteiler erzielt werden, der mit einem Wasser-Kühler oberhalb des Kolonnenkopfes ausgerüstet wurde. Die eingesetzten BPA-Schuppen (Reinheit 99,80 % p,p-BPA) wurden bei einem Rückflußverhältnis von 1,5:1 destilliert.

Unter diesen Bedingungen und nach Abnahme von ca. 30 % Vorlauf, wurde eine Fraktion (44 %) mit einer Reinheit von 99,985 % p,p-BPA-Gehalt isoliert (Bestimmungsmethode: gaschromatographisch nach ASTM-Methode). Die Verunreinigungen wurden mit 10 ppm Phenol, 40 ppm Alkylphenolen, 50 ppm Chromanen und 50 ppm p,p-Me-BPA quantifiziert. Leichtsieder und o,p-BPA wurden im Vorlauf abgetrennt, die Schwersieder Trisphenol und Mol 402 bleiben komplett im Kolonnensumpf zurück.

Die Gewichtsverteilung bei der Flüssigkeitsleiterdestillation sah nach Optimierung wie folgt aus: Vorläufe 10,6% und 22%, Hauptlauf 49,2%; Kolonnenkopfdestillat 7,1%; Kolonnenholdup: 0,8, Sumpf: 9% und Verluste über Vakuumsystem 1,3%.

Die Bilanzierung ergab einen p,p-BPA-Verlust <0,1%.

### Thermo-Farbstabilität von dest-BPA-Schmelze[ nach Bagno-Test]

Die Hauptfraktionen wurden mit Hilfe des Bagno-Tests (166°C, 90 min) auf ihre Thermo-Farbstabilität hin geprüft. Alle Fraktionen zeigten Hazen-Farbzahlen von 5 und <5.

### Bagno-Test

150 g p,p-BPA (fest) wird in 1,5 h in einem nicht inertisierten Glasrohr in einem auf 160°C temperierten Thermostat-Ölbad erhitzt. Das Glasrohr ist nach oben (Öffnung) offen und frei belüftet. Nach 1,5 h wird die Farbe des geschmolzenen BPA an der Hazen-Skala vermessen. Es werden prinzipiell Vergleichstest mit anderen BPA-Schuppen parallel durchgeführt.

### Farbtest BPA-Schuppenware

Destillierte BPA-Schuppenware wurde im Vergleich mit Standard-BPA-Schuppenware (BPA-Reinheiten von 99,63 % bis 99,92 % p,p-BPA) geprüft. Die BPA-Schuppenware wurde 96 h kontinuierlich einer reinen Sauerstoffatmosphäre (500-ml-Glaskolben, 150 g BPA-Schuppen, ca. 100 ml/h kontinuierlich. O₂-Strom, Tageslicht) ausgesetzt. Hierbei wird in Abhängigkeit von der BPA-Reinheit und dem Zeit-/Tageslicht-Einfluß bei allen BPA-Schuppen eine visuell im Vergleich mehr oder minder deutliche Vergilbung der BPA-Schuppen beobachtet. Das destillierte BPA-Material wies auch nach 96 h keine Gelbfärbung auf.

### Messung der Oberflächenspannung/Schaumtest einer NaBPA-Lösung (dest-BPA, krist-BPA, herkömmliches BPA im Vergleich)

Die Oberflächenspannung wurde nach der drop-weight-Methode bestimmt. BPA-Schuppen wurden in einer 14,5%igen NaBPA-Lösung mit einem Hydroxid-Überschuß von 0,2% (selbst festgelegter Standard) eingebracht und gelöst. Bisphenol-A-Schuppenware, herkömmliche Bisphenole, unkristallisierte Bisphenole und destilliertes Bisphenol wurden unter gleichen Bedingungen mit dem Schaumtest geprüft. Destilliertes, hochreines BPA wies die höchsten Werte für die Oberflächenspannung und niedrigsten bei der Schaumhöhenbestimmung.

### Konti-Versuche BPA-Destillation (kontinuierliche Destillation)

Mit Hilfe einer kontinuierlichen, 2-stufigen Destillation wurden Bisphenol-A-Reinheiten von 99,98-99,99% p,p-BPA erreicht (Hazen <5).

### Einstellungen:

### 1. Kolonnenstufe

Zulauf: 400 ml/h; R/E: 0,5; Sumpfabnahme 20 ml/h; Heizmittel Düschi: 250°C

### 2. Kolonnenstufe

R/E: 10; Sumpfabnahme: 360 ml/h; Destillat 20 ml/h; Heizmittel Düschi: 240°C
* = Rücklauf/Entnahme (R/E)

Die Destillation wurde bei einem Betriebsdruck von 2 mbar bis 3 mbar (202°C bis 210°C) durchgeführt. Die Produktzuleitungen sind auf 160°C beheizt, die Kolonneninnentemperaturen liegen ebenfalls bei 160°C. Der adiabate Mantel ist von 250°C zum Vorheizen auf 165°C reduziert worden. Der Schmelzbehälter (Vorlage Destillation) hat eine Temperatur von 200°C. Das BPA-Einsatzprodukt wurde vor dem Einpumpvorgang in die erste Kolonne mit Stickstoff gespült und evakuiert. Bei Nachfüllung der Vorlage sollte eine Pfütze vorliegen.

## Patentansprüche

1. Verfahren zur Herstellung von ultrareinem Bisphenol-A mittels kontinuierlicher oder diskontinuierlicher Destillation, wobei bei diskontinuierlicher Destillation als Kolonnenkopf ein modifizerter Flüssigteiler eingesetzt wird, dadurch gekennzeichnet, daß
(a) eine Bisphenol-A-Schmelze, hergestellt nach üblichen BPA-Herstellungsprozessen oder durch Aufschmelzen von BPA-Schuppen-, Pulver- oder- Prillware, mit einem Gehalt an p,p-Bisphenol A von mindestens 90% und Phenolgehalten <10% als Edukt eingesetzt und destilliert wird,
(b) die Destillation dieser Schmelze ohne Stabilisatorzusatz unter inerten Bedingungen durchgeführt wird,
(c) die Destillation so geführt wird, daß die Verluste durch Zersetzung von p,p-Bisphenol-A <0,5% betragen,
(d) die Destillation bei Temperaturen von 220°C-250°C durchgeführt wird,
(f) die Destillation unter Vakuum bei 1-50 mbar erfolgt,
(g) die Destillation zweistufig erfolgt,
(h) zuerst
(h,1) Schwersiederanteile wie Mol 402, Indane und Trisphenole vollständig abgetrennt werden, dann
(h,2) Metallabrieb, Metallionen- und Salzspuren, Säurespuren, poly-/oligomere Katalysatorausschwemmungen und nicht destillierbaren anderen höhermolekularen Verbindungen, vollständig abgetrennt werden, dann
(h,3) tensidaktive Verbindungen, vollständig abgetrennt werden, dann
(h,4) leichtsiedende Anteile wie o,p-BPA, Alkylphenole, Phenol-, Chromanspuren sowie schwefelhaltige Leichtsieder, wie Mercaptopropionsäure, abgetrennt beziehungsweise stark reduziert werden,
(h,5) mit Leichtsiedern, Phenol, Schwersiedern angereicherte BPA-Schmelzlösungen mit einem Gehalt an p,p-Bisphenol-A >92% als Rückströme in den Prozeß zurückgeführt werden,
(i) die Ausschleusung der mit Leichtsieder angereicherten BPA-Schmelzelösungen in den Prozeß nicht größer als 5% und die Ausschleusung der mit Schwersieder angereicherten BPA-Schmelzelösungen nicht größer als 10% ist,
(j) Leichtsieder und Schwersieder in den mit p,p-BPA vermischten Ausschleuseprodukten aus den Rückströmen in den Prozeß zurückgeführt, umgelagert und teilweise zurückgewonnen werden,
(k) destilliertes p,p-Bisphenol-A als stabiles Sumpfprodukt lastunabhängig auf übliche Weise aufgearbeitet wird.

2. p,p-Bisphenol-A -Schmelze oder -Feststoff, erhältlich nach dem Verfahren nach Anspruch 1 mit einem p,p-BPA-Gehalt > 99,98 Gew.-% und einem o,p-BPA-Gehalt <10 ppm.

3. Verwendung von nach Anspruch 1 gereinigtem p,p-Bisphenol-A als flüssiges, transportierbares und bei Temperaturen >160°C lagerungsfähiges, thermo- und farbstabiles Vorprodukt zur Herstellung von Polymeren.

## Claims

1. Method for the preparation of ultra-pure bisphenol A by means of continuous or discontinuous distillation, a modified liquid separator being used as the column head in the case of discontinuous distillation, characterised in that
(a) a bisphenol A melt, obtained by conventional processes for the preparation of BPA or by melting flakes, powder or pellets of BPA and having a p,p-bisphenol A content of at least 90% and phenol contents of less than 10%, is used as educt and distilled,
(b) the distillation of this melt is carried out without the addition of stabilisers and under inert conditions,
(c) the distillation is carried out in such a way that the losses through decomposition of p,p-bisphenol A are less than 0.5%,
(d) the distillation is carried out at temperatures of from 220°C to 250°C,
(f) the distillation takes place under vacuum at 1 to 50 mbar,
(g) the distillation is carried out in two stages,
(h) first
(h.1) high-boiling components such as Mol 402, indanes and trisphenols are completely separated, then
(h.2) metal dust, metal ions and traces of salt, traces of acid, poly-/oligomeric residues of catalysts and other non-distillable higher molecular compounds are completely separated, then
(h.3) surface-active compounds are completely separated, then
(h.4) low-boiling components such as o,p-BPA, alkylphenols, traces of phenols, traces of chromanes as well as sulfur-containing low-boiling components such as mercaptopropionic acid are separated or greatly decreased,
(h.5) BPA melt solutions enriched with low-boiling components, phenol and high-boiling components and having a p,p-bisphenol A content of greater than 92% are returned to the process as back flows,
(i) not more than 5% of the BPA melt solutions enriched with low-boiling components and not more than 10% of the BPA melt solutions enriched with high-boiling components are channelled into the process,
(j) low-boiling and high-boiling components contained in the channelled products mixed with p,p-BPA are returned to the process from the back flows, rearranged and partially recovered,
(k) distilled p,p-bisphenol A is worked up in a conventional manner as a stable bottom product free of other substances.

2. p,p-bisphenol A melts or solid, obtainable by the method according to claim 1, having a p,p-BPA content of greater than 99.98 wt.% and an o,p-BPA content of less than 10 ppm.

3. Use of p,p-bisphenol A purified according to claim 1 as a liquid intermediate product for the preparation of polymers which is transportable, capable of being stored at temperatures of above 160°C, heat-stable and colourfast.

## Revendications

1. Procédé pour la préparation d'un bisphénol-A purissime par distillation continue ou discontinue avec, dans le cas de la distillation discontinue, un séparateur de liquide modifié en tête de colonne, caractérisé en ce que
(a) on part d'une masse fondue de bisphénol-A préparée par des procédés usuels de préparation ou par fusion d'écailles, de poudres ou de granulés de BPA, à une teneur en p,p-bisphénol-A d'au moins 90 % et des teneurs en phénols <10 %, et on la distille,
(b) on procède à la distillation de cette masse fondue sans addition de stabilisant, dans des conditions inertes,
(c) on conduit la distillation en sorte que les pertes par décomposition du p,p-bisphénol-A soient <0,5 %,
(d) on distille à des températures de 220 à 250°C,
(f) on distille sous un vide de 1 à 50 mbar,
(g) on distille en deux stades opératoires,
(h) en premier lieu
(h,1) on sépare totalement les produits lourds tels que le composé de masse moléculaire 402, les indanes et les trisphénols, puis
(h,2) on sépare totalement les particules métalliques dues à l'abrasion les traces d'ions métalliques et de sels, les traces d'acides, les insolubles contenant des polymères, oligomères et catalyseurs ainsi que les autres composés à haut poids moléculaire non distillables, puis
(h,3) on sépare totalement les composés tensioactifs, puis
(h,4) on sépare totalement ou en fortes proportions les produits légers tels que l'o,p-BPA, les alkylphénols, les traces de phénol et de chromanes et les produits légers contenant du soufre, comme l'acide mercaptopropionique, puis
(h,5) on recycle dans l'opération, en courants de retour, les masses fondues de BPA enrichies en les fractions légères le phénol, les fractions lourdes, à une teneur en p,p-bisphénol-A >92 %,
(i) les quantités de masse fondue de BPA enrichie en produits légers qu'on évacue dans l'opération ne dépassent pas 5 % et les quantités de la masse fondue de BPA enrichie en produits lourds qu'on évacue ne dépassent pas 10 %,
(j) on recycle dans l'opération les produits légers et les produits lods contenus dans les produits évacués en mélange avec du p,p-BPA provenant des courants de retour, on les bloque et on les récupère en partie,
(k) le p,p-bisphénol-A distillé, produit de pied stable, est traité de la manière habituelle, indépendamment de la charge.

2. p,p-bisphénol-A à l'état fondu ou à l'état solide, obtenu par le procédé selon la revendication 1, à une teneur en p,p-BPA > 99,98% en poids et une teneur en o,p-BPA < 10 ppm.

3. Utilisation du p,p-bisphénol-A purifié selon la revendication 1, en tant que matière première liquide, transportable, stable à la chaleur et à coloration stable, stockable à des températures >160°C, pour la fabrication de polymères.
